(19) Europäisches Patentamt
European Patent Office
Office européen des brevets

(11) Numéro de publication: **0 271 556 B1**

(12) **FASCICULE DE BREVET EUROPEEN**

(45) Date de publication du fascicule du brevet: **26.09.90**

(51) Int. Cl.$^5$: **B 01 D 53/14,** B 01 D 53/34

(21) Numéro de dépôt: **87904268.7**

(22) Date de dépôt: **26.06.87**

(86) Numéro de dépôt international: **PCT/FR87/00248**

(87) Numéro de publication internationale: **WO 88/00085 14.01.88 Gazette 88/02**

(54) **PROCEDE ET DISPOSITIF POUR LA DESACIDIFICATION D'UN GAZ RENFERMANT H 2?S OU/ET CO 2? AINSI QUE DES MERCAPTANS.**

(30) Priorité: **30.06.86 FR 8609450**

(43) Date de publication de la demande: **22.06.88 Bulletin 88/25**

(45) Mention de la délivrance du brevet: **26.09.90 Bulletin 90/39**

(84) Etats contractants désignés: **DE GB**

(56) Documents cités:
**EP-A-0 004 043**
**EP-A-0 029 258**
**DE-A-1 935 712**
**US-A-2 524 088**

(73) Titulaire: **SOCIETE NATIONALE ELF AQUITAINE (PRODUCTION)**
**Tour Elf 2, Place de la Coupole La Défense 6**
**F-92400 Courbevoie (FR)**

(72) Inventeur: **BATTEUX, Jacques**
**La Navarrine**
**Serres Morlaas F-64160 Morlaas (FR)**
Inventeur: **SHARONIZADEH, Ahmad**
**Chemin de Lahonde**
**Bernadet F-64160 Morlaas (FR)**

(74) Mandataire: **Boillot, Marc**
**SOCIETE NATIONALE ELF AQUITAINE Division**
**Propriété Industrielle Tour Elf**
**F-92078 Paris la Défense Cédex 45 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 271 556 B1

## Description

L'invention a trait à un procédé pour la désacidification de gaz renfermant $H_2S$ ou/et $CO_2$ ainsi que des mercaptans par absorption de ces composés au moyen d'une solution absorbante régénérable. Elle concerne également un dispositif pour la mise en oeuvre de ce procédé.

L'élimination des composés acides, notamment $H_2S$ ou/et $CO_2$, contenus dans un gaz, encore appelée désacidification dudit gaz, est généralement réalisée par lavage de ce gaz à l'aide d'une solution absorbante qui retient les composés acides par simple dissolution physique ou/et par dissolution après formation d'un sel ou d'un complexe, instable thermiquement, par réaction desdits composés acides avec un composé basique présent dans la solution absorbante.

En pratique, le gaz à traiter, qui renferme les composés acides à éliminer, est mis en contact, dans une zone, dite zone d'absorption, avec la solution absorbante choisie dans des conditions de pression et de température telles que cette solution absorbante fixe la quasi-totalité des composés acides, le gaz épuré sortant en tête de la zone d'absorption. En fond de zone d'absorption, en soutire la solution absorbante chargée des composés acides pour la soumettre à un traitement de régénération, c'est-à-dire pour la débarrasser des composés acides fixés et ainsi restaurer son pouvoir absorbant vis-à-vis desdits composés acides. Pour effectuer cette régénération, on introduit la solution absorbante chargée, soutirée de la zone d'absorption, dans la moitié supérieure d'une zone, dite zone de régénération, et l'on maintient la solution absorbante à régénérer dans cette zone dans des conditions de température et de pression propres à permettre une libération et/ou un entraînement par stripage des composés acides absorbés. Ceci peut être réalisé, en particulier, en maintenant la solution absorbante à régénérer à l'ébullition sous pression dans la zone de régénération, la chaleur nécessaire au maintien de cette ébullition étant fournie par rebouillage de la solution absorbante contenue dans ladite zone, c'est-à-dire par échange indirect de chaleur entre une partie de la solution à régénérer se trouvant dans la moitié inférieure de la zone de régénération et un fluide chaud à température appropriée, généralement de la vapeur d'eau saturée. Au cours de la régénération, les composés acides contenus dans la solution absorbante à régénérer sont libérés et stripés par les vapeurs de la solution absorbante et sont évacués en tête de la zone de régénérataion. En fond de la zone de régénération, on soutire la solution absorbante régénérée et recycle ladite solution régénérée vers la zone d'absorption après avoir éventuellement utilisé cette solution pour réchauffer, par échange indirect de chaleur, la solution à régénérer soutirée de la zone d'absorption avant son introduction dans la zone de régénération.

Lorsque le gaz à désacidifier renferme des mercaptans en plus d'$H_2S$ et/ou $CO_2$, ces mercaptans ne sont que peu ou pas éliminés au cours du lavage du gaz par la solution absorbante. En effet, si les conditions requises pour créer une absorption des mercaptans existent en tête de la zone d'absorption, la solution absorbante en descendant dans ladite zone d'une part se charge en $H_2S$ et $CO_2$ par dissolution physique ou chimique et d'autre part se réchauffe du fait de l'exothermicité des réactions de dissolution. Ces deux phénomènes font que les mercaptans absorbés physiquement en tête de la zone d'absorption sont désorbés et s'échappent pour la plus grande partie dans le gaz traité sortant en tête de la zone d'absorption.

L'invention se propose de remédier à cet inconvénient en fournissant un procédé de désacidification d'un gaz renfermant $H_2S$ et/ou $CO_2$ et également des mercaptans par lavage au moyen d'une solution absorbante régénérable, qui permet non seulement d'éliminer la quasi-totalité des composés acides $H_2S$ et $CO_2$ contenus dans le gaz mais également d'extraire dudit gaz par solubilisation une quantité suffisante de mercaptans, qui permet de satisfaire aux spécifications habituellement requises pour le gaz traité.

Le procédé suivant l'invention pour la désacidification d'un gaz renfermant $H_2S$ et/ou $CO_2$ et également des mercaptans, est du type dans lequel on met en contact le gaz à traiter avec un liquide absorbant régénérable, circulant à contre-courant, dans une zone d'absorption primaire, de manière à produire un gaz à teneur prédéterminée fortement réduite en $H_2S$ et $CO_2$ et un courant primaire de liquide absorbant chargé d'$H_2S$ et $CO_2$ et l'on soumet ledit courant primaire de liquide absorbant à une régénération, dans une zone de régénération, pour libérer les composés acides absorbés, qui sont évacués en tête de la zone de régénération, et produire en fond de zone de régénération un liquide absorbant régénéré primaire que l'on soutire et recycle vers la zone primaire d'absorption et il se caractérisé en ce que l'on prélève une fraction du liquide absorbant régénéré primaire recyclé et introduit ladite fraction dans la partie supérieure d'une zone d'absorption secondaire en fond de laquelle on injecte le gaz issu de la zone primaire d'absorption, en ce que l'on recueille le gaz épuré en tête de la zone secondaire d'absorption et l'on soutire en fond de ladite zone secondaire un courant secondaire du liquide absorbant chargé de mercaptans et soumet ledit courant à une réduction de pression, dans une zone de détente, de manière à désorber les mercaptans qu'il contient et à former un liquide absorbant régénéré secondaire substantiellement exempt de mercaptans et en ce que l'on réunit ledit absorbant régénéré secondaire au liquide absorbant régénéré primaire avant de prélever sur ce dernier la fraction dirigée vers la zone secondaire d'absorption.

Le procédé suivant l'invention peut être utilisé pour la désacidification de tous types de gaz renfermant $H_2S$ et/ou $CO_2$ ainsi qu'une proportion de mercaptans pouvant aller jusqu'à quelques pour cent en volume et par exemple jusqu'à 4%

en volume ou plus, qui sont disponibles sous des pressions allant d'environ 10 bars absolus à environ 100 bars absolus. En particulier, un tel procédé est bien adapté pour le traitement des divers gaz, naturels ou non, renfermant $H_2S$ et/ou $CO_2$ et des mercaptans en vue de produire des gaz épurés ayant une teneur globale minimale fixée en soufre total.

Le liquide absorbant utilisable pour la mise en oeuvre du procédé suivant l'invention peut être choisi parmi les divers liquides absorbants qui, d'une part, sont susceptibles de fixer $H_2S$ et $CO_2$ et de libérer ensuite lesdits composés sous l'action d'un chauffage et/ou d'une détente et, d'autre part, sont aptes à dissoudre les mercaptans et ensuite à libérer ces produits sous l'action d'une détent éventuellement combinée à un réchauffage. En particulier le liquide absorbant peut consister en une solution aqueuse d'un ou plusieurs composés basiques et notamment en une solution aqueuse d'une ou plusieurs alcanolamines primaires ou secondaires telles que monoéthanolamine, diéthanolamine, diisopropanolamine, alcanolamine primaire de formule

$$HO—C_2H_4—O—C_2H_4—NH_2$$

connue sous nom de DIGLYCOLAMINE®.

Le liquide absorbant peut également consister en un mélange d'une solution aqueuse d'un ou plusieurs composés basiques et notamment d'une ou plusieurs alcanolamines primaire ou secondaire telles que celles précitées avec un ou plusieurs solvants organiques, par exemple sulfolane ou méthanol. Convient tout spécialement comme liquide absorbant dans le procédé suivant l'invention, une solution aqueuse d'une ou plusieurs alcanolamines primaires ou secondaires, par example monoéthanolamine, diéthanolamine, diisopropanolamine ou diglycolamine, dont la concentration globale en alcanolamine est comprise entre 1N et 8N et se situe de préférence entre 3N et 6N.

La pression régnant dans chacune des zones d'absorption primaire et secondaire correspond, à la perte de charge près, à celle du gaz à traiter que l'on injecte dans la zone d'absorption primaire et peut donc aller d'environ 10 bars absolus à environ 100 bars absolus.

La température du liquide absorbant introduit dans la zone d'absorption primaire dépend, entre autres, de la nature dudit liquide absorbant et du point de rosée du gaz sortant en tête de ladite zone. Par exemple, lorsque le liquide absorbant consiste en une solution aqueuse d'alcanolamine, la température dudit liquide absorbant au point d'introduction dans la zone d'absorption peut être comprise entre 10°C et 80°C et de préférence entre 20°C et 60°C.

Le débit de liquide absorbant, qui circule dans la zone d'absorption primaire à contre-courant du gaz à traiter, est lié, entre autres, à la teneur en composés acides $H_2S$ et $CO_2$ du gaz à traiter et également à la quantité globale de gaz acides que l'on tolère dans le gaz traité sortant en tête de ladite zone d'absorption primaire. Le débit de liquide absorbant introduit dans la zone d'absorption primaire est ajusté pour extraire la quasitotalité des composés acides $H_2S$ et $CO_2$ présente dans le gaz à traiter.

Les conditions de pression et de température imposées dans la zone de régénération du liquide absorbant primaire sont choisies, compte-tenu de la nature dudit liquide absorbant, de telle sorte que les composés gazeux acides $H_2S$ et $CO_2$ retenus par le liquide absorbant durant son passage dans la zone d'absorption primaire soient libérés et que le liquide absorbant soutiré en fond de la zone de régénération soit pratiquement exempt de composés gazeux dissous. La pression absolue en tête de la zone de régénération est généralement comprise entre 1 et 5 bars et se situe le plus souvent entre 1,3 et 2,5 bars. Dans le cas d'une régénération du liquide absorbant chargé de composés gazeux acides par rebouillage, le maintien d'une telle pression impose une température en fond de zone de régénération comprise habituellement entre 100°C et 150°C environ, ce qui correspond à une température en tête de la zone de régénération allant d'environ 80°C à 125°C.

La température de la fraction de liquide absorbant introduite dans la zone d'absorption secondaire est égale ou légèrement inférieure à celle du liquide absorbant introduit dans la zone d'absorption primaire et telle que l'absorption des mercaptans, par ladite fraction soit quasi-complète de telle sorte que le gaz triaté évacué en tête de la zone d'absorption secondaire ait une teneur en soufre total répondant aux spécifications imposées. De préférence, la température de ladite fraction est inférieure à 50°C lorsque le liquide absorbant est une solution aqueuse d'alcanolamine.

Le débit de la fraction de liquide absorbant introduite dans la zone d'absorption secondaire peut varier assez largement et représente avantageusement 20% à 100% et de préférence 30% à 60% du débit de liquide absorbant introduit dans la zone d'absorption primaire.

Dans la zone de détente, le courant secondaire de liquide absorbant chargé de mercaptans, qui est soutiré de la zone d'absorption secondaire, est détendu pour réduire sa pression de telle sorte que la quasi-totalité des mercaptans dissous soit libérée. En fond de la zone de détente on soutire un courant secondaire de liquide absorbant substantiellement exempt de mercaptans que l'on réunit au liquide absorbant régénéré issue de la zone de régénération tandis qu'en tête de la zone de détente on évacue un gaz chargé de mercaptans. Avantageusement le courant secondaire de liquide absorbant chargé de mercaptans est réchauffé avant d'être introduit dans la zone de détente. De préférence, ce réchauffage est réalisé pour augmenter d'environ 10°C à 50°C la température du courant secondaire de liquide absorbant avant son introduction dans la zone de détente.

Préalablement à son introduction dans la zone de régénération, le liquide absorbant primaire

chargé des composés acides $H_2S$ et $CO_2$ peut être soumis à une detent en vue de libérer les gaz et notamment les hydrocarbures qu'il a pu dissoudre physiquement lors de son contact avec le gaz à traiter. Les gaz libérés lors de cette détente peuvent être utilisés comme gaz combustibles dans des installations de chauffage ou être envoyés à la torche. Le gaz chargé de mercaptans, évacué de la zone de détente recevant le courant de liquide absorbant secondaire, peut être réuni aux gaz libérés lors de la détente du courant primaire de liquide absorbant issu de la zone d'absorption primaire.

Un dispositif pour la mise en oeuvre du procédé suivant l'invention est, comme le dispositif connu par EP—A—029258, du type comportant:

—une colonne primaire d'absorption, munie un tête d'une sortie pour les gaz et en fond d'une sortie pour les liquides et équipée, dans sa partie inférieure, d'un conduit d'injection du gaz à traiter et, dans sa partie supérieure, d'une amenée de liquide absorbant,

—une colonne secondaire d'absorption présentant en tête une sortie pour les gaz, cette dernière se prolongeant par un conduit, et en fond une sortie pour les liquides, ladite colonne secondaire étant munie dans sa partie inférieure d'une amenée de gaz, reliée à la sortie pour les gaz en tête de la colonne primaire d'absorption, et dans sa partie supérieure d'une amenée de liquide absorbant alimentée par le système de recyclage,

—et une colonne de régénération pourvue en tête d'un conduit d'évacuation pour les gaz et équipée, à sa partie supérieure, d'une amenée de liquide absorbant, qui est connectée à la sortie pour les liquides en fond de la colonne d'absorption primaire, à sa partie inférieure, d'un système de chauffage et en fond d'une sortie pour les liquides, ladite sortie étant connectée, à travers un système de recyclage, à l'amenée de liquide absorbant de la colonne primaire d'absorption.

Il est caractérisé en ce que l'alimentation en liquide absorbant de la colonne secondaire d'absorption est faite à travers un système de refroidissement et en ce que la sortie pour les liquides de la colonne secondaire d'absorption est connectée par un conduit à un orifice d'un ballon de détente comportaur en tête un orifice pour les gaz, prolongé par un conduit d'évacuation, en fond un orifice de sortie pour les liquides prolongé par un conduit de soutirage amenant le liquide qu'il transporte au système de recyclage.

Avantageusement la colonne primaire d'absorption et la colonne secondaire d'absorption forment deux sections superposées d'une même colonne d'absorption, la colonne secondaire constituant la section supérieure de cette colonne d'absorption unique, lesdites sections communiquant par un passage pour les gaz débouchant dans la section supérieure, qui joue le rôle de colonne secondaire, au-dessus de la sortie pour les liquides aménagée en fond de cette colonne secondaire.

Le système de recyclage de liquide absorbant est avantageusement constitué d'un bac de stockage présentant une ou plusieurs entrées connectant ledit bac à la sortie pour les liquides en fond de la colonne de régénération à travers un conduit équipé de préférence d'un système de refroidissement, au conduit de soutirage associé au ballon de détente et éventuellement à une amenée de liquide absorbant d'appoint, ainsi qu'une sortie reliée par un conduit à l'amenée de liquide absorbant à la colonne primaire d'absorption, ledit conduit portant une dérivation munie d'un système de refroidissement par échange indirect de calories et reliée à l'amenée de liquide absorbant dans la colonne secondaire d'absorption, des moyens étant prévus sur les conduits arrivant au bac de stockage et partant de ce dernier pour assurer la circulation du liquide absorbant.

De préférence, le conduit reliant la sortie pour les liquides de la colonne secondaire d'absorption au ballon de détente est équipé d'un système de réchauffage, de tout type connu, placé soit en amont soit en aval de la vanne de régulation de pression montée sur ledit conduit. En particulier, ledit système de réchauffage peut consister en un échangeur indirect de chaleur dont le circuit froid est monté en série sur le conduit reliant la sortie pour les liquides de la colonne secondaire d'absorption au ballon de détente et le circuit chaud est placé en série sur le conduit reliant la sortie pour les liquides en fond de la colonne de régénération au bac de stockage formant le système de recyclage ou sur une dérivation montée sur ledit conduit.

La connexion entre la sortie pour les liquides en fond de la colonne primaire d'absorption et l'amenée de liquide absorbant présente dans la partie supérieure de la colonne de régénération peut être réalisée à travers un ballon d'expansion, qui comporte en tête une sortie pour les gaz prolongée par un conduit d'évacuation de gaz et en fond une sortie pour les liquides connectée par un conduit à l'amenée de liquide de la colonne de régénération, ledit ballon d'expansion présentant en outre une entrée pour les liquides reliée par un conduit à la sortie pour les liquides en fond de la colonne primaire d'absorption. Dans ce cas, le conduit d'évacuation de gaz associé au ballon de détente est de préférence connecté au conduit d'évacuation de gaz associé au ballon d'expansion.

Chacune des colonnes d'absorption et de régénération, qui font partie du dispositif suivant l'invention, peut être de tout type connu habituellement utilisé pour la mise en contact d'un gaz avec un liquide et peut par exemple consister en une colonne à plateaux ou encore en une colonne à garnissage.

Le nombre de plateaux ou la hauteur équivalente de garnissage des colonnes utilisées est choisi pour qu'en fonctionnement chacune des colonnes joue correctement son rôle de manière à obtenir le degré d'épuration souhaité du gaz à traiter et le niveau de régénération désiré du liquide absorbant chargé des impuretés acides.

L'invention sera mieux comprise à la lecture de

la description suivante de l'une de ses formes de mise en oeuvre illustrée par la figure du dessin annexé représentant un dispositif suivant l'invention utilisant des colonnes à plateaux.

En se référant à la figure, le dispositif pour la désacidification d'un gaz renfermant $H_2S$ et/ou $CO_2$ ainsi que des mercaptans comporte deux colonnes, à savoir une colonne 1 d'absorption et une colonne 2 de régénération, qui sont chacune équipées de plateaux de contact gaz/liquide. La colonne 1 est constituée de deux sections d'absorption superposées, à savoir une section 3 inférieure, dite section primaire d'absorption, et une section 4 supérieure, dite section secondaire d'absorption, lesdites sections communiquant par un passage 5 pour les gaz. Une sortie 6 pour les gaz est prévue en tête de la colonne 1, c'est-à-dire en tête de la section secondaire d'absorption, ladite sortie étant prolongée par un conduit 7 d'évacuation des gaz, tandis qu'une sortie 8 pour les liquides est prévue en fond de la colonne 1, c'est-à-dire en fond de la section primaire d'absorption. Dans sa partie inférieure, c'est-à-dire dans la partie inférieure de la section primaire d'absorption, la colonne 1 est équipée d'un conduit 9 d'injection du gaz à traiter. Ladite colonne 1 comporte encore, à la partie supérieure de chacune des sections primaire et secondaire d'absorption, une amenée, respectivement 10 et 11, de liquide absorbant tandis qu'une sortie 12 pour les liquides est prévue en fond de la section secondaire d'absorption.

La colonne 2 de régénération est pourvue en tête d'une sortie 13 pour les gaz se prolongeant par un conduit 14 d'évacuation des gaz et en fond d'une sortie 15 pour le liquide absorbant régénéré. Dans sa partie supérieure la colonne 2 de régénération est munie d'une amenée 16 de liquide absorbant à régénérer tandis que dans sa partie inférieure ladite colonne est associée, par des tubulures d'entrée 17 et de sortie 18, à un rebouilleur 19 chauffé par échange indirect de calories au moyen de vapeur d'eau saturée circulant dans une tubulaire 20.

La sortie 8 pour les liquides de la collone 1 est connectée, par un conduit 21, pourvu d'une vanne 21a de régulation de pression, à l'entrée 22 pour les liquides d'un ballon d'expansion 23. Ce ballon d'expansion est pourvu d'une sortie 24 pour les gaz prolongée par un conduit 25 d'évacuation des gaz et d'une sortie 26 pour les liquides, ladite sortie 26 étant connectée par un conduit 27, à travers le circuit froid d'un échangeur indirect de chaleur 28, à l'amenée 16 de liquide absorbant à régénérer de la colonne 2 de régénération.

La sortie 15 pour les liquides de la colonne 2 de régénération est connectée par un conduit 29, à travers le circuit chaud de l'échangeur de chaleur 28 puis à travers un système de refroidissement 30, à une première entrée 31 d'un bac 32 de stockage de liquide absorbant, ledit bac possédant une seconde entrée 33 et une sortie 34. Cette sortie est reliée, par un conduit 35 sur lequel est montée une pompe 36, à l'amenée 10 de liquide absorbant de la section primaire d'absorption 3 de la colonne 1. Un conduit 37, monté en dérivation sur le conduit 35 en aval de la pompe 36, est connecté, à travers un système de refroidissement 38, à l'amenée 11 de liquid absorbant de la section secondaire d'absorption 4 de la colonne 1. La sortie 12 pour les liquides de ladite section secondaire d'absorption 4 est reliée à l'entrée 40 pour les liquides d'un ballon 41 de détente par un conduit 39 équipé d'une vanne 46 de régulation de pression et sur lequel est monté en série le circuit froid d'un échangeur indirect de chaleur 48 dont le circuit chaud est placé en série sur un conduit 49, qui est monté en dérivation sur le conduit 29 en amont du système de refroidissement 30. Le ballon 41 est pourvu, en outre, d'une sortie 42 pour les gaz, prolongée par un conduit 43 d'évacuation des gaz relié au conduit 25, et d'une sortie 44 pour les liquides, connectée, par un conduit 45 équipé d'un système de refroidissement 47, à la deuxième entrée 33 du bac de stockage.

Le fonctionnement de ce dispositif peut être schématisé comme suit:

Le gaz à traiter, par exemple un gaz naturel, qui renferme les composés acides à extraire, à savoir $H_2S$ et/ou $CO_2$, ainsi que des mercaptans, arrive sous une pression élevée, par exemple de l'ordre de 40 à 80 bars absolus, par le conduit 9 dans la section primaire d'absorption 3 de la colonne 1 et rencontre, à contre-courant, le liquide absorbant régénérable par chauffage, qui est introduit dans ladite section primaire 3, par l'amenée 10 de liquide absorbant en provenance du bac de stockage 32, et s'écoule par gravité dans la section primaire d'absorption 3. Dans cette section d'absorption, le liquide absorbant fixe la quasi-totalité des composés acides $H_2S$ et $CO_2$ et seulement une faible quantité des mercaptans présents dans le gaz à traiter. Ledit gaz, arrivant à la partie supérieure de la section primaire d'absorption 3, pénètre à travers le passage 5 dans la section secondaire d'absorption 4, dans laquelle il entre en contact, à contre-courant, avec la fraction de liquide absorbant arrivant par le conduit 37, à travers le système de refroidissement 38, et introduite dans ladite section secondaire d'absorption 4 par l'amenée 11 de liquide absorbant. La température de cette fraction de liquide absorbant est amenée, lors de son passage dans le système de refroidissement 38, à une valeur inférieure à la température du courant de liquide absorbant injecté dans la section primaire d'absorption 3 et telle que ladite fraction refroidie de liquide absorbant, au cours de sa descente par gravité dans la section secondaire d'absorption 4, fixe par dissolution la quantité désirée des mercaptans présents dans le gaz en cours de traitement. Par la sortie 6 de la colonne 1, c'est-à-dire en tête de la section secondaire d'absorption 4, sort un gaz épuré que l'on évacue par le conduit 7, ledit gaz ayant des teneurs résiduelles en composés acides $H_2S$ et $CO_2$ et en mercaptans abaissées aux valeurs minimales désirées.

Le liquide absorbant chargé des composés gazeux acides $H_2S$ et $CO_2$ et d'une petite quantité

de mercaptans absorbés dans la section primaire d'absorption 3 de la colonne 1, sort de ladite colonne par la sortie 8 et arrive, par le conduit 21, dans le ballon d'expansion 23, dans lequel la pression dudit liquide absorbant est abaissée avec comme résultat la libération d'une phase gazeuse formée des composés gazeux, notamment hydrocarbures et mercaptans, dissous physiquement par le liquide absorbant, cette phase gazeuse étant évacuée par le conduit 25. Le courant de liquide absorbant chargé des composés acides $H_2S$ et $CO_2$, dont la pression a été réduite dans le ballon d'expansion 23, est alors amené à la colonne de régénération 2, par le conduit 27, après réchauffage dans l'échangeur de chaleur 28. Dans la colonne de régénération le liquide absorbant est mainteu à l'ébullition sous une pressin supérieure à la pression atmosphérique et généralement comprise entre 1 et 5 bars absolus, de manière à libérer les composés gazeux acides absorbés et à réaliser leur stripage par les vapeurs du liquide absorbant. Le liquide àbsorbant régénéré est soutiré de la colonne de régénération 2 par la sortie 15 en fond de colonne et recyclé dans le bac de stockage 32 par les conduits 29 et 49, sous l'action d'une pompe non représentée, après avoir été refroidi à la température appropriée pour l'absorption primaire par passage dans les échangeurs de chaleur 28 et 48, pour transférer des calories respectivement au liquide absorbant à régénérer amené par le conduit 27 à la colonne de régénération et au courant secondaire de liquide absorbant chargé de mercaptans, puis dans le système de refroidissement 30. Les calories nécessaires au maintien à l'ébullition du liquide absorbant dans la colonne de régénération sont fournies par passage d'une partie du liquide régénéré, soutiré par la sortie 15, dans le rebouilleur 19 chauffé par de la vapeur d'eau saturée passant dans la tubulure 20 et retour du liquide absorbant chaud à la colonne de régénération par la tubulure 18.

Les composés gazeux acides $H_2S$ et $CO_2$ libérés dans la colonne de régénération sont stripés par les vapeurs du liquide absorbant et sortent en tête de ladite colonne par la sortie 13 pour être évacués par le conduit 14.

Le courant secondaire de liquide absorbant chargé des mercaptans fixés par dissolution physique dans la section secondaire d'absorption 4, sort de cette section secondaire par la sortie 12 en fond de ladite section puis est amené, par le conduit 39, dans le ballon 41 de détente en subissant un réchauffage dans l'échangeur de chaleur 48. Dans ce ballon 41 la pression du liquide est abaissée suffisamment pour que la quasi-totalité des mercaptans dissous par le liquide absorbant soit libérée, lesdits mercaptans étant évacués du ballon 41 par la sortie 42 et le conduit 43 et réunis à la phase gazeuse évacuée par le conduit 25. Le liquide absorbant substantiellement exempt de mercaptans est soutiré du ballon 41 par la sortie 44 et ramené, par le conduit 45 avec refroidissement dans le système 47 de refroidissement, au bac de stockage 32 dans lequel ledit liquide absorbant est mélangé au liquide absorbant régénéré issu de la colonne 2 de régénération.

Pour compléter la description de l'invention qui vient d'être présentée, on donne ci-après, à titre non limitatif, un exemple concret de mise en oeuvre du procédé suivant l'invention.

Exemple

On traitait un gaz naturel consistant en majeure partie en méthane et renfermant, exprimés en volume, 0,5% d'$H_2S$ et 5% de $CO_2$ ainsi que 200 mg/$Nm^3$ de méthyl mercaptan et d'éthylmercaptan à titre d'impuretés, en opérant dans un dispositif analogue à celui décrit en référence à la figure du dessin annexé.

Dans le dispositif utilisé la colonne d'absorption comportait une section primaire d'absorption et une section secondaire d'absorption renfermant respectivement 20 et 5 plateaux et la colonne de régénération comportait 21 plateaux.

Le liquide absorbant consistait en une solution aqueuse de diéthanolamine (en abrégé DEA) renfermant 3 moles de DEA par litre.

Le gaz naturel à traiter arrivait dans la section primaire d'absorption 3, par le conduit 9, avec un débit de 62 000 $Nm^3$/h, une pression absolue de 75 bars et une température d'environ 35°C et rencontrait dans ladite section, à contre-courant, la solution aqueuse de DEA introduite dans la section primaire d'absorption 3, par l'amenée 10 de liquide absorbant, avec un débit de 60 $m^3$/h et une température d'environ 40°C.

Dans cette section primaire d'absorption la quasi-totalité des composés acides $H_2S$ et $CO_2$ était fixée par la solution aqueuse de DEA, cette solution n'absorbant par contre dans cette zone qu'une très faible partie des mercaptans.

Arrivé à la partie supérieure de la section primaire d'absorption 3, le gaz en cours de traitement pénétrait dans la section secondaire d'absorption 4 et rencontrait, à contre-courant, la fraction de solution aqueuse de DEA introduite dans ladite section d'absorption, par l'amenée 11 de liquide absorbant, avec un débit de 30 $m^3$/h et une température d'environ 37°C.

Dans cette section secondaire d'absorption la quasi-totalité des mercaptans était fixée par la solution aqueuse refroidie de DEA, cette solution fixant également les quantités résiduelles d'$H_2S$ et de $CO_2$ présentes dans le gaz.

Par la sortie 6 de la colonne 1, on évacuait un gaz naturel désacidifié renfermant en volume 2,5 p.p.m. d'$H_2S$ et 100 p.p.m. de $CO_2$ ainsi que 80 mg/$Nm^3$ de mercaptans.

La solution aqueuse de DEA était portée en fond de la colonne de régénération à une température d'environ 125°C, par de la vapeur d'eau saturée sous une pression absolue de 4 bars circulant dans la tubulure 20 du rebouilleur 19, de manière à maintenir une pression absolue de 2,2 bars en tête de ladite colonne de régénération.

Par la sortie 13 en tête de la colonne de régénération 2 on évacuait 2 300 $Nm^3$/h d'un flux gazeux acide formé des gaz acides libérés au

cours de la régénération de la solution de DEA et renfermant en volume 14% d'$H_2S$ et 85% de $CO_2$, le reste consistant en vapeur d'eau.

La réduction de pression de la solution aqueuse de DEA chargée d'$H_2S$ et de $CO_2$ dans le ballon d'expansion 23 libérait 50 Nm³/h d'une phase gazeuse consistant essentiellement en méthane, ladite phase étant évacuée par le conduit 25.

Par la sortie 12 de la section secondaire d'absorption 4 on soutirait 30 m³/h de solution de DEA chargée de mercaptans, ladite solution ayant une température d'environ 37°C et circulant dans le conduit 39 sous une pression de 74,5 bars absolus, et on amenait ce courant de solution, après réchauffage à environ 60°C dans l'échangeur de chaleur 48, dans le ballon 41 de détente, dans lequel la pression dudit courant de solution était abaissée à une valeur d'environ 2,5 bars absolus. La détente de la solution libérait la quasi-totalité des mercaptans dissous, que l'on évacuait par la sortie 42 du ballon 41 et le conduit d'évacuation 43. Par le conduit 45 on soutirait la solution aqueuse de DEA pratiquement débarrassée des mercaptans et amenait ladite solution, avec un débit de 30 m³/h, au bac de stockage 32 en vue de sa réutilisation, ladite solution ayant une température d'environ 40°C à sa sortie du système 47 de refroidissement.

Dans un essai comparatif, on traitait le gaz naturel ayant la composition précitée, en opérant dans des conditions comparables à celles définies ci-dessus avec toutefois remplacement de la colonne d'absorption 1 à deux sections primaire et secondaire d'absorption par une colonne d'absorption à section unique d'absorption comportant 25 plateaux, injection unique de la solution avec un débit de 90 m³/h et une température d'environ 40°C et régénération de la totalité de la solution aqueuse de DEA, chargée des composés acides absorbés, dans la colonne de régénération 2 fonctionnant par rebouillage.

Le gaz traité sortant en tête de la colonne d'absorption renfermait, en volume, 2,5 p.p.m. d'$H_2S$ et 100 p.p.m. de $CO_2$ ainsi que 180 mg/Nm³ de mercaptans.

## Revendications

1. Procédé pour la désacidification d'un gaz renfermant $H_2S$ et/ou $CO_2$ et également des mercaptans, du type dans lequel on met en contact le gaz à traiter avec un liquide absorbant régénérable, circulant à contre-courant, dans une zone primaire d'absorption, de manière à produire un gaz à teneur réduite en $H_2S$ et $CO_2$ et un courant primaire de liquide absorbant chargé d'$H_2S$ et de $CO_2$ et l'on soumet ledit courant primaire de liquide absorbant à une régénération, dans une zone de régénération, pour libérer les composés acides $H_2S$ et $CO_2$ absorbés, qui sont évacués en tête de la zone de régénération, et produire en fond de zone de régénération un liquide absorbant régénéré primaire que l'on soutire et recycle vers la zone primaire d'absorption et se caractérisant en ce que l'on prélève une fraction du liquide absorbant régénéré primaire recyclé et introduit ladite fraction dans la partie supérieure d'une zone d'absorption secondaire en fond de laquelle on injecte le gaz issu de la zone primaire d'absorption, en ce que l'on recueille le gaz épuré en tête de la zone secondaire d'absorption et l'on soutire en fond de ladite zone secondaire un courant secondaire de liquide absorbant chargé de mercaptans et soumet ledit courant à une réduction de pression, dans une zone de détente, de manière à désorber les mercaptans qu'il contient et à former un liquide absorbant régénéré secondaire substantiellement exempt de mercaptans et en ce que l'on réunit ledit liquide absorbant régénéré secondaire au liquide absorbant régénéré primaire avant de prélever sur ce dernier la fraction dirigée vers la zone secondaire d'absorption.

2. Procédé suivant la revendication 1, caractérisé en ce que la température de la fraction de liquide absorbant introduite dans la zone d'absorption secondaire est inférieure à celle du liquide absorbant introduit dans la zone d'absorption primaire.

3. Procédé suivant la revendication 1 ou 2, caractérisé en ce que le gaz à traiter renferme une proportion de mercaptans allant jusqu'à quelques pour cent et notamment jusqu'à 4% en volume.

4. Procédé suivant la revendication 1 ou 2 ou 3, caractérisé en ce que le gaz à traiter a une pression allant d'environ 10 bars absolus à environ 100 bars absolus.

5. Procédé suivant l'une des revendications 1 à 4, caractérisé en ce que le liquide absorbant consiste en une solution aqueuse d'une ou plusieurs alcanolamines primaires ou secondaires, dont la concentration globale en alcanolamine est comprise entre 1N et 8N et de préférence entre 3N et 6N.

6. Procédé suivant la revendication 5, caractérisé en ce que la température de la fraction de liquide absorbant introduite dans la zone secondaire d'absorption est inférieure à 50°C.

7. Procédé suivant l'une des revendications 1 à 6, caractérisé en ce que le débit de la fraction de liquide absorbant introduite dans la zone secondaire d'absorption représente 20% à 100% et de préférence 30% à 60% du débit de liquide absorbant introduit dans la zone primaire d'absorption.

8. Procédé suivant l'une des revendications 1 à 7, caractérisé en ce que le courant secondaire de liquide absorbant chargé des mercaptans, que l'on soutire de la zone secondaire d'absorption, est réchauffé avant d'être introduit dans la zone de détente.

9. Procédé suivant la revendication 8, caractérisé en ce que ledit réchauffage est réalisé pour augmenter d'environ 10°C à 50°C la température du courant secondaire de liquide absorbant chargé de mercaptans avant l'introduction dudit courant dans la zone de détente.

10. Dispositif pour la désacidification d'un gaz renfermant $H_2S$ et/ou $CO_2$ ainsi que des mercaptans, du type comportant:

—une colonne (3) primaire d'absorption, munie en tête (5) d'une sortie pour les gaz et en fond d'une sortie (8) pour les liquides et équipée, dans sa partie inférieure, d'un conduit (9) d'injection du gaz à traiter et, dans sa partie supérieure, d'une amenée (10) de liquide absorbant,

—une colonne (4) secondaire d'absorption présentant en tête une sortie (6) pour les gaz, cette dernière se prolongeant par un conduit (7), et en fond une sortie (12) pour les liquides, ladite colonne secondaire étant munie dans sa partie inférieure d'une amenée (5) de gaz, reliée à la sortie pour les gaz en tête de la colonne primaire d'absorption, et dans sa partie supérieure d'une amenée (11) de liquide absorbant alimentée par le système de recyclage,

—et une colonne (2) de régénération pourvue en tête (13) d'un conduit (14) d'évacuation pour les gaz et équipée, à sa partie supérieure, d'une amenée (16) de liquide absorbant, qui est connectée à la sortie (8) pour les liquides en fond de la colonne d'absorption primaire (3), à sa partie inférieure, d'un système de chauffage (19, 20) et en fond d'une sortie (15) pour les liquides, ladite sortie (15) étant connectée, à travers un système de recyclage (29, 32, 35), à l'amenée (10) de liquide absorbant de la colonne primaire d'absorption (3)

caractérisé en ce que l'alimentation en liquide absorbant de la colonne secondaire d'absorption est faite à travers un système de refroidissement (38) et en ce que la sortie (12) pour les liquides de la colonne secondaire d'absorption est connectée par un conduit (39) à un orifice (40) d'un ballon de détente (41) comportaur en tête un orifice (42) pour les gaz, prolongé par un conduit d'évacuation (43), en fond un orifice (44) de sortie pour les liquides prolongé par un conduit (45) de soutirage amenant le liquide qu'il transporte au système de recyclage (32).

11. Dispositif suivant la revendication 10, caractérisé en ce que la colonne primaire d'absorption (3) et la colonne secondaire d'absorption (4) forment deux sections superposées d'une même colonne d'absorption (1), la colonne secondiare d'absorption constituant la section supérieure de cette colonne (1) d'absorption et en ce que lesdites sections communiquent par un passage (5) pour les gaz, qui débouche dans la section (4) jouant le rôle de colonne secondaire d'absorption au-dessus de la sortie (12) pour les liquides située en fond de ladite section.

12. Dispositif suivant la revendication 10 ou 11, caractérisé en ce que le système de recyclage est constitué d'un bac (32) de stockage présentant une ou plusieurs entrées (31, 33) connectant ledit bac à la sortie (15) pour les liquides en fond de la colonne (2) de régénération à travers un conduit (29) de préférence équipé d'un système (30) de refroidissement, au conduit (45) de soutirage associé au ballon (41) de détente et éventuellement à une amenée de liquide absorbant d'appoint ainsi qu'un sortie (34) reliée par un conduit (35) à l'amenée (10) de liquide absorbant dans la colonne (3) primaire d'absorption, ledit conduit (35) portant une dérivation (37) munie d'un système (38) de refroidissement et reliée à l'amenée (11) de liquide absorbant dans la colonne (4) secondaire d'absorption, des moyens (36) étant prévus sur les conduits arrivant au bac (32) de stockage et partant de ce dernier pour assurer la circulation du liquide absorbant.

13. Dispositif suivant l'une des revendications 10 à 12, caractérisé en ce que le conduit (39) reliant la sortie pour les liquides de la colonne (4) secondaire d'absorption au ballon de détente (41) est équipé d'un système de réchauffage (48).

14. Dispositif suivant la revendication 13, caractérisé en ce que ledit système de réchauffage (48) consiste en un échangeur indirect de chaleur dont le circuit froid est monté en série sur le conduit (39) et le circuit chaud est placé en série sur le conduit (29) reliant la sortie (15) pour les liquides en fond de la colonne (2) de régénération au bac (32) de stockage ou sur un conduit (49) monté en dérivation sur ledit conduit (29).

15. Dispositif suivant l'une des revendications 10 à 13, caractérisé en ce qu'il inclut un ballon (23) d'expansion, qui comporte en tête une sortie (24) pour les gaz prolongée par un conduit (25) d'évacuation des gaz et en fond une sortie (26) pour les liquides connectée par un conduit (27), éventuellement à travers le circuit froid d'un échangeur indirect de chaleur (28) dont le circuit chaud est placé en série sur le conduit (29) reliant la sortie (15) pour les liquides en fond de la colonne (2) de régénération au bac (32) de stockage, à l'amenée (16) de liquide dans la colonne de régénération, ledit ballon (23) d'expansion présentant en outre une entrée (22) pour les liquides reliée, par un conduit (21) pourvu d'une vanne (21a) de régulation de pression, à la sortie (8) pour les liquides en fond de la colonne (3) primaire d'absorption.

16. Dispositif suivant la revendication 15, caractérisé en ce que le conduit d'évacuation de gaz (43) associé au ballon (41) de détente est connecté au conduit (25) d'évacuation de gaz associé au ballon (23) d'expansion.

**Patentansprüche**

1. Verfahren zur Entsäuerung eines $H_2S$ und/oder $CO_2$ sowie Merkaptane enthaltenden Gases, bei dem man das zu behandelnde Gas in einer ersten Absorptionszone mit einer regenerierbaren absorbierenden, im Gegenstrom fließenden Flüssigkeit so kontaktiert, daß man ein Gas mit einem verminderten $H_2S$- und $CO_2$-Gehalt und einen ersten Strom der mit $H_2S$ und $CO_2$ beladenen Absorptionsflüssigkeit erzeugt und diesen ersten Strom der Absorptionsflüssigkeit in einer Regenerationszone einer Regeneration unterzieht, um die absorbierten sauren Verbindungen $H_2S$ und $CO_2$ freizusetzen, die am Kopf der Regenerationszone abgelassen werden, und am Boden dieser Zone eine erste regenerierte Absorptionsflüssigkeit zu erzeugen, die man abzieht und der ersten Absorptionszone wieder zuführt, dadurch gekennzeichnet, daß man eine Fraktion der zurückgeführten ersten regenerierten Absorptionsflüssigkeit abzieht und dem oberen Teil einer zweiten Absorptionszone zuführt, an deren Boden das aus der ersten Absorptionszone stammende Gas eingespritzt wird und daß man das am Kopf der zweiten Absorptionszone gereinigte Gas sammelt und man am Boden der zweiten Zone einen zweiten Strom der mit Merkaptanen beladenen Absorptionsflüssigkeit abzieht und diesen Strom in einer Entspannungszone einer Druckminderung unterzieht, um die darin enthaltenen Merkaptane zu desorbieren und eine zweite, im wesentlichen von Merkaptanen freie regenerierte Absorptionsflüssigkeit zu bilden und daß man diese zweite regenerierte Absorptionsflüssigkeit mit der ersten regenerierten Absorptionsflüssigkeit vereinigt, bevor man der zuletzt genannten die in Richtung der zweiten Absorptionszone geführte Fraktion entnimmt.

2. Verfahren nach Anspruch 1, dadurch gekennzeichnet, daß die Temperatur der Fraktion der der zweiten Absorptionszone zugeführten Absorptionsflüssigkeit unterhalb derjenigen der der ersten Absorptionszone zugeführten Absorptionsflüssigkeit liegt.

3. Verfahren nach Anspruch 1 oder 2, dadurch gekennzeichnet, daß das zu behandelnde Gas eine Anteil an Merkaptanen von bis zu einigen Volumenprozent und insbesondere bis zu 4 Volumenprozent enthält.

4. Verfahren nach Anspruch 1 oder 2 oder 3, dadurch gekennzeichnet, daß das zu behandelnde Gas einen Druck von ca. 10 bis ca. 100 bar aufweist.

5. Verfahren nach einem der Ansprüche 1 bis 4, dadurch gekennzeichnet, daß die Absorptionsflüssigkeit aus einer wäßrigen Lösung eines oder mehrerer primärer oder sekundärer Alkanolamine besteht, deren Gesamtalkanolaminkonzentration zwischen 1 und 8 N und vorzugsweise zwischen 3 und 6 N beträgt.

6. Verfahren nach Anspruch 5, dadurch gekennzeichnet, daß die Temperatur der Fraktion der der zweiten Absorptionszone zugeführten Absorptionsflüssigkeit unter 50°C liegt.

7. Verfahren nach einem der Ansprüche 1 bis 6, dadurch gekennzeichnet, daß die Durchflußmenge der Fraktion der der zweiten Absorptionszone zugeführten Absorptionsflüssigkeit 20 bis 100% und vorzugsweise 30 bis 60% der Durchflußmenge der der ersten Absorptionszone zugeführten Absorptionsflüssigkeit beträgt.

8. Verfahren nach einem der Ansprüche 1 bis 7, dadurch gekennzeichnet, daß der zweite Strom der mit Merkaptanen beladenen Absorptionsflüssigkeit, den man aus der zweiten Absorptionszone abzieht, vor der Zufuhr der Entspannungszone erneut erwärmt wird.

9. Verfahren nach Anspruch 8, dadurch gekennzeichnet, daß die Wiederarwärmung durchgeführt wird, um die Temperatur des zweiten Stroms der mit Merkaptanen beladenen Absorptionsflüssigkeit vor der Zufuhr des Stroms zur Entspannungszone um ca. 10 bis 50°C zu erhöhen.

10. Vorrichtung zur Entsäuerung eines $H_2S$ und/oder $CO_2$ sowie Merkaptane enthaltenden Gases, wobei diese Vorrichtung

—eine erste Absorptionskolonne (3), die am Kopf (5) mit einer Austrittsöffnung für die Gase und am Boden mit einer Austrittsöffnung (8) für die Flüssigkeiten sowie in ihrem unteren Teil mit einer Leitung (9) zum Einspritzen des zu behandelnden Gases und in ihrem oberen Teil mit einer Leitung (10) für die Zuführ der Absorptionsflüssigkeit ausgestattet ist,

—eine zweite Absorptionskolonne (4), die am Kopf einen Auslaß (6) für die Gase aufweist, der sich in der Leitung (7) fortsetzt, und am Boden einen Auslaß (12) für die Flüssigkeiten, wobei diese zweite Kolonne in ihrem unteren Teil mit einer Leitung (5) für die Gaszufuhr ausgestattet ist, die mit dem Auslaß für die Gase am Kopf der ersten Absorptionskolonne verbunden ist, und in ihrem oberen Teil mit einer Leitung (11) für die Zufuhr der über das Rückführungssystem zugespeisten Absorptionsflüssigkeit,

—und eine Regenerationskolonne (2), die an ihrem Kopf (13) mit einer Leitung (14) für die Ableitung der Gase versehen ist, und in ihrem oberen Teil mit einer Leitung (16) für die Zufuhr der Absorptionsflüssigkeit, die mit dem Auslaß (8) für die Flüssigkeiten am Boden der ersten Absorptionskolonne (3) verbunden ist, in ihrem unteren Teil mit einem Heizsystem (19, 20), und am Boden mit einem Auslaß (15) für die Flüssigkeiten ausgestattet ist, wobei dieser über ein Rückführungssystem (29, 32, 35) mit der Leitung (10) für die Zufuhr der Absorptionsflüssigkeit der ersten Absorptionskolonne verbunden ist, umfaßt, dadurch gekennzeichnet, daß die Zuspeisung der Absorptionsflüssigkeit der zweiten Absorptionskolonne über ein Kühlsystem (38) erfolgt und der Auslaß (12) für die Flüssigkeiten der zweiten Absorptionskolonne über eine Leitung (39) mit der Eintrittsöffnung (40) eines Entspannungsbehälters (41) verbunden ist, der am Kopf eine Öffnung (42) für den Austritt der Gase aufweist, die sich in eine Ausströmleitung (43) fortsetzt, und am Boden eine Öffnung (44) für den Austritt der Flüssigkeiten, die sich in eine Abzugsleitung (45) fortsetzt, über die die Flüssigkeit in das Rückführungssystem (32) geleitet wird.

11. Vorrichtung nach Anspruch 10, dadurch gekennzeichnet, daß die erste Absorptionskolonne (3) und die zweite Absorptionskolonne (4) zwei übereinander angeordnete Abschnitte ein und derselben Absorptionskolonne (1) bilden, wobei die zweite Absorptionskolonne den oberen Abschnitt der Absorptionskolonne (1) darstellt und die Abschnitte miteinander durch einen Kanal (5) für den Durchtritt der Gase verbunden sind, der in den Abschnitt (4), welcher die Rolle der zweiten Absorptionskolonne spielt, oberhalb des am Boden des genannten Abschnitts angeordneten Austritts (12) für die Flüssigkeiten mündet.

12. Vorrichtung nach Anspruch 10 oder 11, dadurch gekennzeichnet, daß das Rückführungssystem aus einem Speicherbehälter (32) besteht,

der einen oder mehrere Eintrittsöffnungen (31, 33), die diesen mit dem Austritt (15) für die Flüssigkeiten am Boden der Regenerationskolonne (2) über eine Leitung (29), die vorzugsweise mit einem Kühlsystem (30) ausgestattet ist, mit der Abzugsleitung (45), die mit dem Entspannungsbehälter (41) verbunden ist, und gegebenenfalls mit einer zusätzlichen Leitung für die Zufuhr der Absorptionsflüssigkeit verbinden sowie eine Austrittsöffnung (34) aufweist, die über eine Leitung (35) mit der Leitung (10) für die Zufuhr der Absorptionsflüssigkeit zur ersten Absorptionskolonne (3) verbunden ist, wobei die Leitung (35) eine Abzweigung (37) aufweist, die mit einem Kühlsystem (38) ausgestattet und mit der Leitung (11) für die Zufuhr der Absorptionsflüssigkeit zur zweiten Absorptionskolonne (4) verbunden ist, und an den Leitungen, die zum Speicherbehälter (32) führen und von diesem wegführen, Mittel (36) vorgesehen sind, um den Kreislauf der Absorptionsflüssigkeit zu gewährleisten.

. 13. Vorrichtung nach einem der Ansprüche 10 bis 12, dadurch gekennzeichnet, daß die den Austritt für die Flüssigkeiten aus der zweiten Absorptionskolonne (4) mit dem Entspannungsbehälter (41) verbindende Leitung (39) mit einem Heizsystem (48) ausgestattet ist.

14. Vorrichtung nach Anspruch 13, dadurch gekennzeichnet, daß das Heizsystem (48) aus einem indirekten Wärmetauscher besteht, dessen kalter Kreislauf mit der Leitung (39) in Serie geschaltet ist und dessen warmer Kreislauf mit der Leitung (29), welche den Austritt (15) für die Flüssigkeiten am Boden der Regenerationskolonne (2) mit dem Speicherbehälter (32) verbindet, oder mit einer mit der Leitung (29) parallel geschalteten Leitung (49) in Serie geschaltet ist.

15. Vorrichtung nach einem der Ansprüche 10 bis 13, dadurch gekennzeichnet, daß sie einen Ausdehnungsbehälter (23) umfaßt, der am Kopf einen Austritt (24) für die Gase, der sich in einer Leitung (25) für die Ableitung der Gase fortsetzt, und am Boden einen Austritt (26) für die Flüssigkeiten aufweist, der über eine Leitung (27), gegebenenfalls über den kalten Kreislauf eines indirekten Wärmetauschers (28), dessen warmer Kreislauf mit der Leitung (29) in Serie geschaltet ist, welche den Austritt (15) für die Flüssigkeiten am Boden der Regenerationskolonne (2) mit dem Speicherbehälter (32) verbindet, mit der Leitung (16) für die Zufuhr der Flüssigkeit zur Regenerationskolonne verbunden ist, wobei der Ausdehnungsbehälter (23) außerdem eine Eintrittsöffnung (22) für die Flüssigkeiten aufweist, die über eine mit einem Druckregelventil (21a) versehene Leitung (21) mit der Austrittsöffnung (8) für die Flüssigkeiten am Boden der ersten Absorptionskolonne (3) verbunden ist.

16. Vorrichtung nach Anspruch 15, dadurch gekennzeichnet, daß die mit dem Entspannungsbehälter (41) verbundene Leitung (43) für die Ableitung der Gase mit der mit dem Ausdehnungsbehälter (23) in Verbindung stehenden Leitung (25) für die Ableitung der Gase verbunden ist.

**Claims**

1. A process for the deacidification of a gas containing $H_2S$ and/or $CO_2$ and also mercaptans, of the type in which the gas to be treated is brought into contact with a regenerable absorbent liquid circulating in counter-current in a primary absorption zone in order to produce a gas with a reduced content of $H_2S$ and $CO_2$ and a primary flow of absorbent liquid charged with $H_2S$ and $CO_2$ and in which the said primary flow of absorbent liquid is subjected to a regeneration stage in a regeneration zone in order to release the absorbed $H_2S$ and $CO_2$ compounds which are drawn off at the head of the regeneration zone and in order to produce at the bottom of the regeneration zone a primary regenerated absorbent liquid which is drawn off and recycled to the primary absorption zone, the process being characterised in that a fraction of the recycled primary regenerated absorbent liquid is drawn off, the said fraction being introduced into the upper part of a secondary absorption zone into the bottom of which is injected the gas emanating from the primary absorption zone and in that the purified gas is collected at the head of the secondary absorption zone, a secondary flow of absorbent liquid charged with mercaptans being drawn off at the bottom of the said secondary zone and subjected to a reduction in pressure in an expansion zone in order to desorb the mercaptans which it contains and in order to form a substantially mercaptan-free secondary regenerated absorbent liquid and in that the said secondary regenerated absorbent liquid is combined with the primary regenerated absorbent liquid before drawing off from this former the fraction which is directed to the secondary absorption zone.

2. A process according to claim 1, characterised in that the temperature of the fraction of absorbent liquid which is introduced into the secondary absorption zone is lower than that of the absorbent liquid which is introduced into the primary asborbent zone.

3. A process according to claim 1 or 2, characterised in that the gas to be treated contains a proportion of mercaptans which may extend up to a few percent and in particular up to 4% by volume.

4. A process according to claim 1 or 2 or 3, characterised in that the gas to be treated is at a pressure ranging from about 10 bars absolute to about 100 bars absolute.

5. A process according to one of claims 1 to 4, characterised in that the absorbent liquid consists of an aqueous solution of one or a plurality of primary or secondary alkanolamines of which the total alkanolamine concentration is comprised between 1 N and 8 N and is preferably between 3 N and 6 N.

6. A process according to claim 5, characterised in that the temperature of the fraction of absorbent liquid which is introduced into the secondary absorption zone is below 50°C.

7. A process according to one of claims 1 to 6, characterised in that the rate of flow of the

absorbent liquid fraction introduced into the secondary absorption zone represents 20% to 100% and preferably 30% to 60% of the rate of flow of absorbent liquid introduced into the primary absorption zone.

8. A process according to one of claims 1 to 7, characterised in that the secondary flow of mercaptan charged absorbent liquid which is drawn off from the secondary absorption zone is reheated before being introduced into the expansion zone.

9. A process according to claim 8, characterised in that the said reheating is carried out in order to raise by about 10°C to 50°C the temperature of the secondary flow of mercaptan charged absorbent liquid before the said flow is introduced into the expansion zone.

10. An apparatus for deacidifying a gas containing $H_2S$ and/or $CO_2$ and also mercaptans of the type comprising:

—a primary absorption column (3) provided with a gas outlet at the top (5) and a liquids outlet (8) at the bottom and equipped in its lower part with a pipe (9) for the injection of gas to be treated and in its upper part with an absorbent liquid supply means (10),

—a secondary absorption column (4) having at the top a gas outlet (6), this latter being extended by a pipe (7) and having at the bottom a liquids outlet (12), the said secondary column being provided in its lower part with a gas supply means (5) connected to the gas outlet at the top of the primary absorption column and having in its upper part supply means (11) for absorbent liquid supplied by the recycling system,

—and a regeneration column (2) provided at the top (13) with a gas discharge pipe (14) and equipped in its upper part with absorbent liquid supply means (16) connected to the liquids outlet (8) at the bottom of the primary absorption column (3), in its lower part with a heating system (19, 20) and at the bottom with a liquids outlet (15) which is connected via a recycling system (29, 32, 35) to the absorbent liquid supply means (10) on the primary absorption column (3), characterised in that the supply of absorbent liquid to the secondary absorption column is carried out via a cooling system (38) and in that the liquids outlet (12) from the secondary absorption column is connected by a pipe (39) to an orifice (40) of an expansion vessel (41) comprising at the top a gas orifice (42) extended by a discharge pipe (43) and having at the bottom an orifice (44) serving as an outlet for liquids and extended by a draw-off pipe (45) feeding liquid which it conveys to the recycling system (32).

11. An apparatus according to claim 10, characterised in that the primary absorption column (3) and the secondary absorption column (4) form two superposed sections of one and the same absorption column (1), the secondary absorption column constituting the upper section of this absorption column (1) and in that the said sections communicate by a gas passage (5) which discharges into the section (4) serving as a secondary absorption column above the liquids outlet (12) situated at the bottom of the said section.

12. An apparatus according to claim 10 or 11, characterised in that the recycling system consists of a storage tank (32) having one or a plurality of inlets (31, 33) connecting the said tank to the liquids outlet (15) at the bottom of the regeneration column (2) via a pipe (29) preferably equipped with a cooling system (30), to the draw-off pipe (45) associated with the expansion vessel (41) and possibly to means of supplying top-up absorbent liquid and an outlet (34) connected by a pipe (35) to the absorbent liquid supply means (10) in the primary asborption column (3), the said pipe (35) carrying a by-pass (37) provided with a cooling system (38) and connected to the absorbent liquid supply means (11) in the secondary absorption column (4), means (36) being provided on the pipes arriving at the storage tank (32) and leaving this latter in order to ensure the circulation of absorbent liquid.

13. An apparatus according to one of claims 10 to 12, characterised in that the pipe (39) connecting the liquids outlet of the secondary absorption column (4) to the expansion vessel (41) is equipped with a reheating system (48).

14. An apparatus according to claim 13, characterised in that the said reheating system (48) consists of an indirect heat exchanger, the cold circuit of which is mounted in series on the pipe (39), the hot circuit being placed in series on the pipe (29) connecting the liquids outlet (15) at the bottom of the regeneration column (2) to the storage tank (32) or on a pipe (49) mounted as a by-pass on the said pipe (29).

15. An apparatus according to one of claims 10 to 13, characterised in that it includes an expansion vessel (23) comprising at the top a gas outlet (24) extended by a gas discharge pipe (25) and having at the bottom a liquids outlet (26) connected by a pipe (27) possibly via the cold circuit of an indirect heat exchanger (28), of which the hot circuit is mounted in series on the pipe (29) connecting the liquids outlet (15) at the bottom of the regeneration column (2) to the storage tank (32), to the liquids supply means (16) to the regeneration column, the said expansion vessel (23) having, furthermore, a liquids inlet (22) connected by a pipe (21) provided with a pressure regulating valve (21a) to the liquids outlet (8) at the bottom of the primary absorption column (3).

16. An apparatus according to claim 15, characterised in that the gas discharge pipe (43) associated with the expansion vessel (41) is connected to the gas discharge pipe (25) associated with the expansion vessel (23).

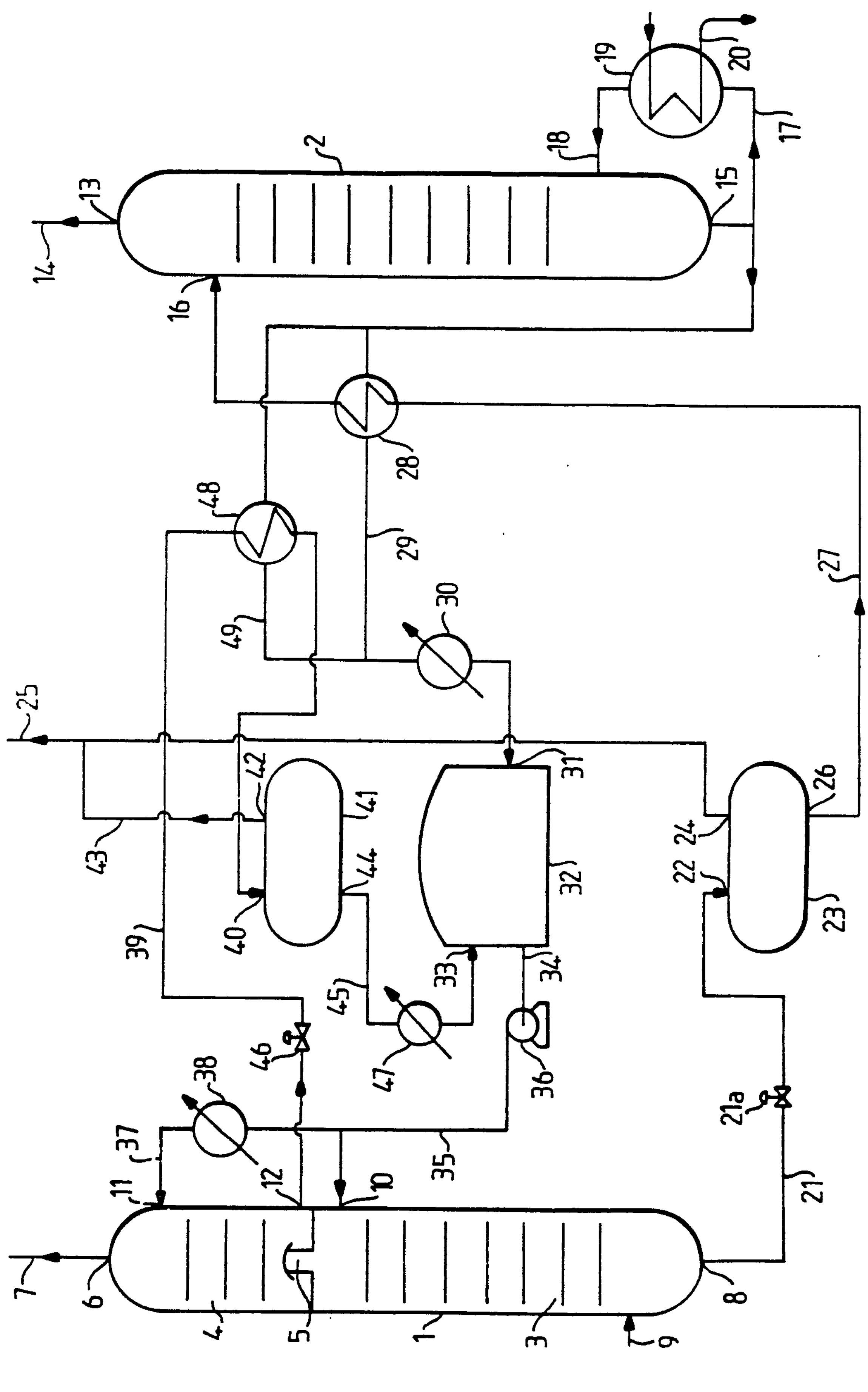
1
2
3
4
5
6
7
8
9
10
11
12
13
14
15
16
17
18
19
20
21
21a
22
23
24
25
26
27
28
29
30
31
32
33
34
35
36
37
38
39
40
41
42
43
44
45
46
47
48
49